# EUROPEAN PATENT APPLICATION

(11) **EP 2 703 030 A2**
(43) Date of publication of application: **05.03.2014**
(21) Application number: 13163508.8
(22) Date of filing: 12.04.2013
(51) Int. Cl.: A61M 11/06, A61M 16/14, A61M 16/20

(54) **Medical atomizer**

(30) Priority: 31.08.2012 CN 201220443597 U
(71) Applicant: Rossmax International LTD., Taipei, 114 (TW)
(72) Inventor: Wang, Chih-Hua, Taipei 114 (TW)
(74) Representative: Ter Meer Steinmeister & Partner

(57) **Abstract**

A medical atomizer includes a liquid storage container (100), a nozzle (200) and an adjustable valve (300). The liquid storage container (100) includes an air inlet (121), a fixed cover plate installed at the periphery of the air inlet, and a high pressure jet (111) installed at the bottom of the liquid storage container (100). The nozzle (200) is installed in the liquid storage container (100) and covered onto the high pressure jet (111). The adjustable valve (300) is interconnected to the air inlet (121) and has a port (310) selectively adjusted and moved to be covered by fixed cover plate (123). With the fixed cover plate (123) of the air inlet (121), the air inlet (121) can be selectively adjusted and moved to change the size of the port (310) to be covered by the fixed cover plate (120), so as to adjust the flow of air entering into the air inlet (121).

## Description

### FIELD OF THE INVENTION

The present invention relates to a medical atomizer, and more particularly to the medical atomizer with an adjustable medicine nebulization rate.

### BACKGROUND OF THE INVENTION

At present, oral medication is the most popular way of taking medicine, but the oral medication has the drawback that some medicines may be depleted in a patient's digestive tract before entering into the patient's stomach, so that oral medication is not suitable for medicines of this sort. To overcome this drawback, aerosol medication is provided as an alternative for patients to take medicine through the patients' respiratory system. For example a medical atomizer produces aerosol medicine by a nebulizer and provides the aerosol medicine for the patients to inhale through the patients' mouth or noise and further into the patients' trachea, bronchus, and alveolus. Obviously, the aerosol medicine has a better and more efficient and direct effect than the oral medicine.

In general, a conventional atomizer structure comprises a cup shaped body, a nozzle, a cap, a high pressure jet installed in the cup shaped body, a spray outlet formed on the cap or body, and a liquid medicine stored in the cup shaped body. By injecting high pressure air into the cup shaped body, the air passes through the high pressure jet into the cup shaped body, and then the air is mixed and atomized with the liquid medicine around the top opening of nozzle, and finally the aerosol medicine is passed through the spray outlet and provided for an inhale by a patient.

The atomizer may be used with different air pressure sources. If a different air pressure source is used, the nebulization rate of the medicine is also different. Different patients (such as adults and children) require different dosages (or different nebulization rates). In addition, the viscosities of different medicines are different, so that the efficiency for the atomizer to atomize different medicines varies.

The conventional atomizer is unable to adjust the nebulization rate for different applications and results in low medication efficiency and high medical fees.

### SUMMARY OF THE INVENTION

Therefore, it is a primary objective of the present invention to provide a medical atomizer with an adjustable medicine nebulization rate.

To achieve the aforementioned objective, the present invention provides a medical atomizer comprising a liquid storage container, a nozzle and an adjustable valve. The liquid storage container includes an air inlet, a fixed cover plate installed at the periphery of the air inlet, and a high pressure jet installed at the bottom of the liquid storage container. The nozzle is installed in the liquid storage container and covered onto the high pressure jet. The adjustable valve is interconnected to the air inlet and has a port selectively adjusted and moved to be covered by fixed cover plate.

Preferably, the liquid storage container of the medical atomizer comprises a main body and a cap installed to the main body, and the air inlet is formed on the cap.

Preferably, the medical atomizer further comprises a duct with an end interconnected to the air inlet.

Preferably, the duct and the cap of the medical atomizer are integrally formed as a whole.

Preferably, the duct of the medical atomizer has a baffle extended from an outer side of the duct.

Preferably, the baffle of the duct is tilted towards the bottom of the liquid storage container.

Preferably, another end of the duct of the medical atomizer is covered onto the nozzle.

Preferably, the liquid storage container of the medical atomizer has a spray outlet formed thereon.

Preferably, the cap of the medical atomizer has a spray outlet formed thereon.

Preferably, the nozzle of the medical atomizer has a liquid injection opening formed at the tip of the nozzle.

In the present invention, the adjustable valve is provided for adjusting the flow of natural air to control the nebulization rate of the liquid medicine to cope with different dosages for different medication conditions and achieve the effects of avoiding unnecessary waste of medical resources and overcoming the drawbacks of the prior art.

### BRIEF DESCRIPTION OF THE DRAWINGS

FIG. 1 is a perspective view of a medical atomizer of a preferred embodiment of the present invention;

FIG. 2 is an exploded view of a medical atomizer of a preferred embodiment of the present invention;

FIG. 3 is a cross-sectional view of a medical atomizer of a preferred embodiment of the present invention;

FIG. 4 is a schematic view of a closed adjustable valve of a medical atomizer of a preferred embodiment of the present invention;

FIG. 5 is a schematic view of a fully opened adjustable valve of a medical atomizer of a preferred embodiment of the present invention;

FIG. 6 is a schematic view, showing the operation of a closed adjustable valve of a medical atomizer of a preferred embodiment of the present invention;

FIG. 7 is a schematic view, showing the operation of a fully opened adjustable valve of a medical atomizer of a preferred embodiment of the present invention.

### DESCRIPTION OF THE PREFERRED EMBODIMENTS

The technical contents of the present invention will become apparent with the detailed description of preferred embodiments accompanied with the illustration of related drawings as follows.

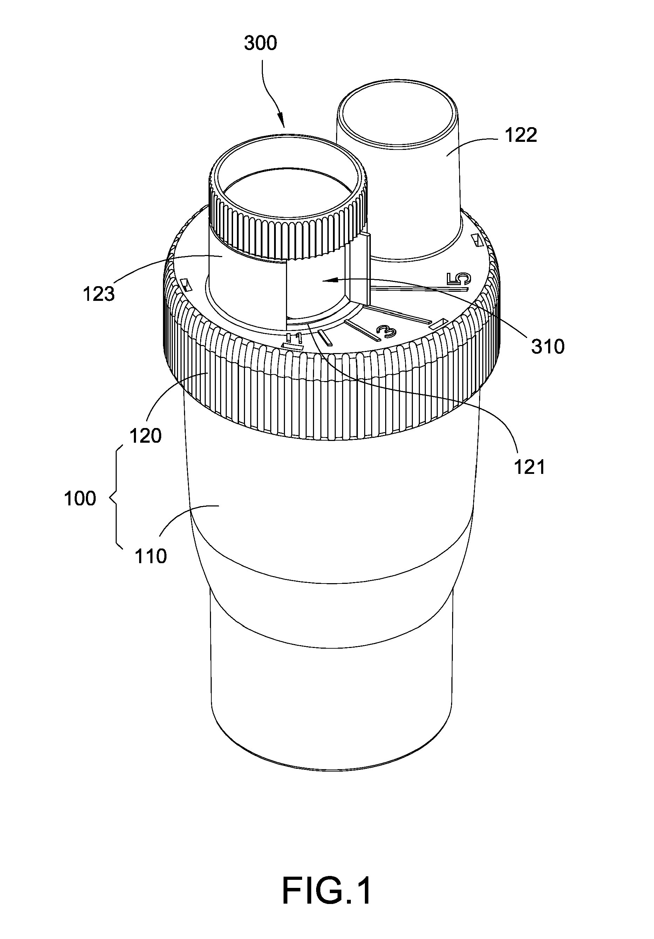

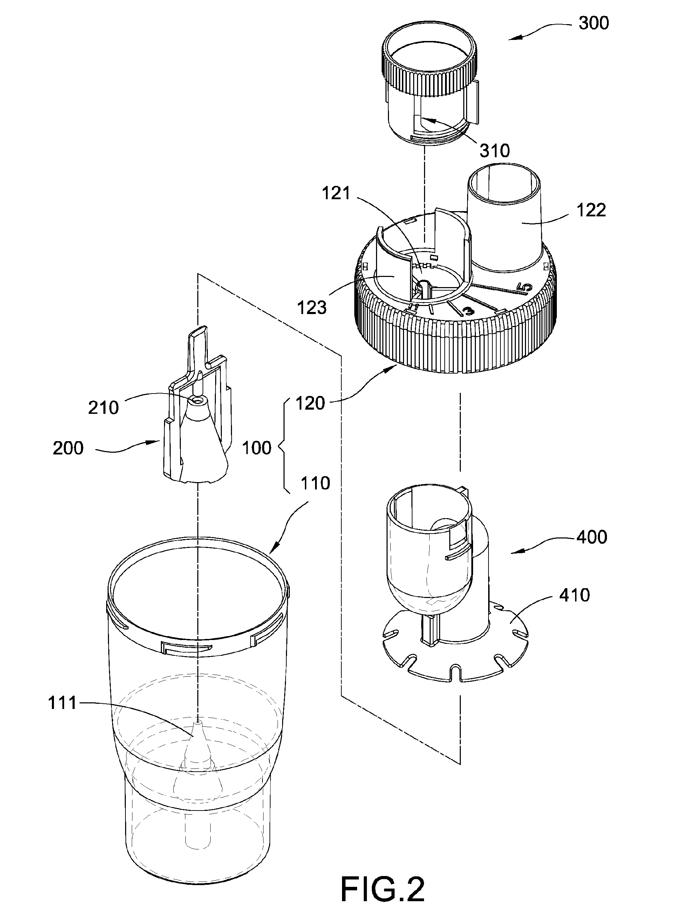

With reference to FIGS. 1 and 2 for a medical atomizer of a preferred embodiment of the present invention, liquid medicine is mixed with air to produce atomized medicine as a medication for patients, and the medical atomizer comprises a liquid storage container 100, a nozzle 200, an adjustable valve 300 and a duct 400.

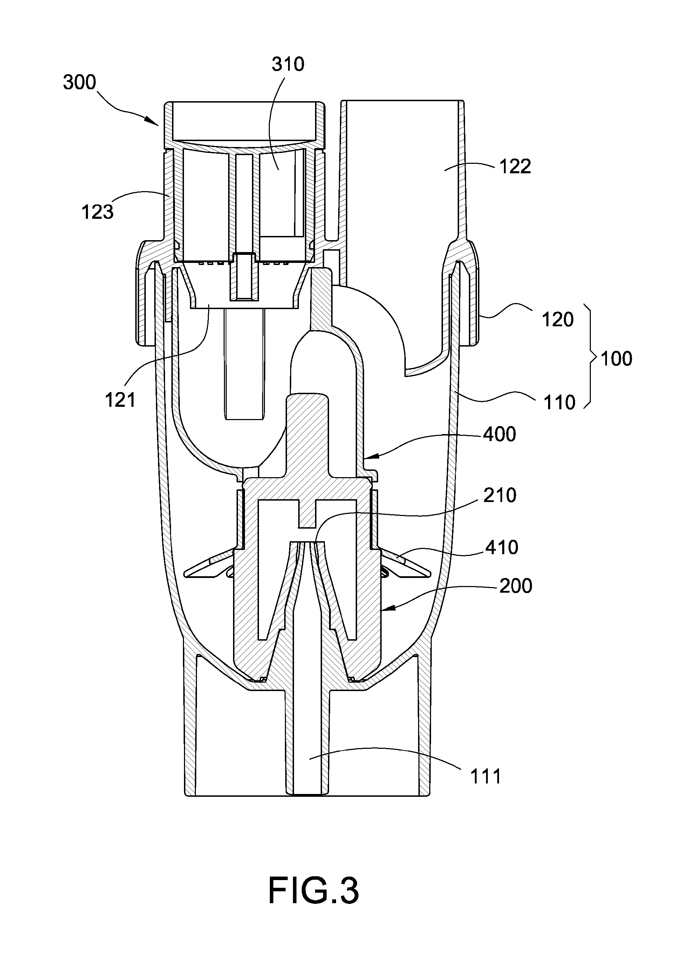

In FIGS. 2 and 3, the liquid storage container 100 comprises a main body 110 and a cap 120. The main body 110 is substantially cup shaped, and the bottom of cup shaped body 110 is provided for containing the liquid medicine. A high pressure jet 111 is installed on an inner side of the bottom of the main body 110, and the high pressure jet 111 is in a conical shape and used for filling high pressure air into the main body 110. The cap 120 is covered onto the main body 110 and has an air inlet 121 and a spray outlet 122, wherein the air inlet is provided for flowing natural air outside the liquid storage container 100 into the liquid storage container 100, and the spray outlet 122 is provided for patients to inhale the atomized medicine contained in the liquid storage container 100.

The nozzle 200 is sheathed on the high pressure jet 111, and a liquid injection opening 210 is formed at the tip of the nozzle 200.

The duct 400 can be a standalone component or integrally formed with the cap 120. The duct 400 is installed in the main body 110, and an end of the duct 400 is covered onto the nozzle 200 and the other end of the duct 400 is interconnected to the air inlet 121. A baffle 410 is extended from an outer side of the duct 400 and preferably disposed at an end of the duct 400 proximate to the nozzle 200 and the baffle 410 is substantially ring-shaped and arranged around the duct 400 and tilted towards the bottom of the liquid storage container 100.

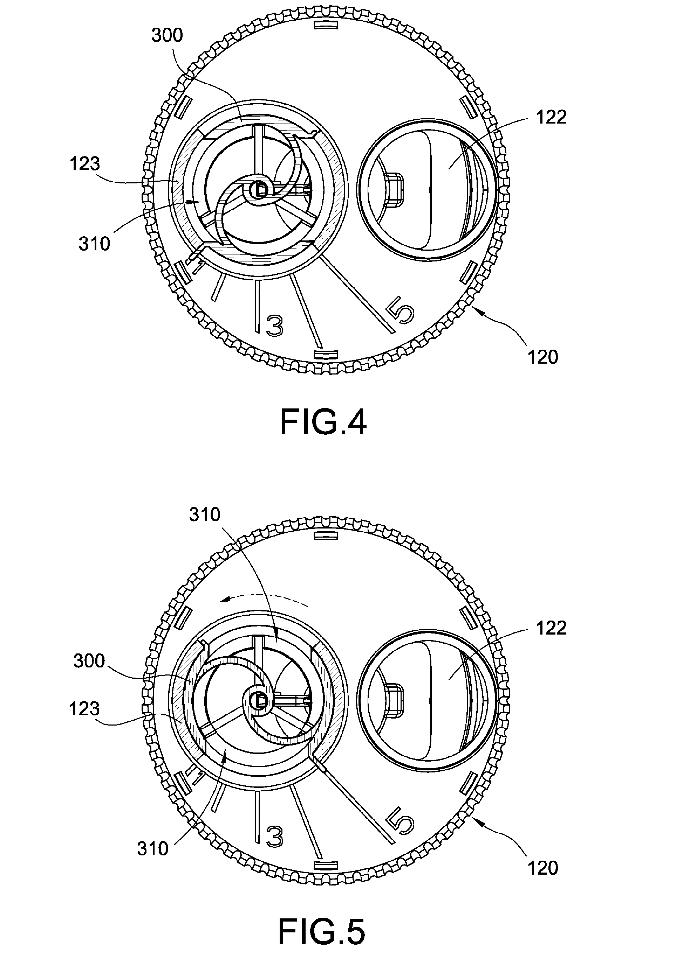

In FIGS. 2 and 4, the adjustable valve 300 is installed on an outer side of the cap 120 and interconnected to the air inlet 121. The adjustable valve 300 is substantially a cylindrical body with at least one port 310 formed on a sidewall of the cylindrical body. In this preferred embodiment, there are two ports 310, and the ports 310 are formed on a side of the adjustable valve 300 to provide a greater flow area than the top surface. In this preferred embodiment, the quantity of ports 310 is preferably two, so that the two ports 310 can be covered by the two fixed cover plate 123 of the cap 120 respectively, and the two fixed cover plates 123 are installed at the periphery of the air inlet 121, wherein the two fixed cover plates 123 are preferably circular arc plates, and the fixed cover plates 123 are sheathed on the adjustable valve 300, so that the adjustable valve 300 can be rotated with the center of the cylindrical body and moved along the inner wall of the fixed cover plate 123. By rotating the adjustable valve 300 to move the port 310, the area for the two fixed cover plates 123 to cover the two ports 310 can be changed. In other words, the size of the port interconnected to the outside can be changed.

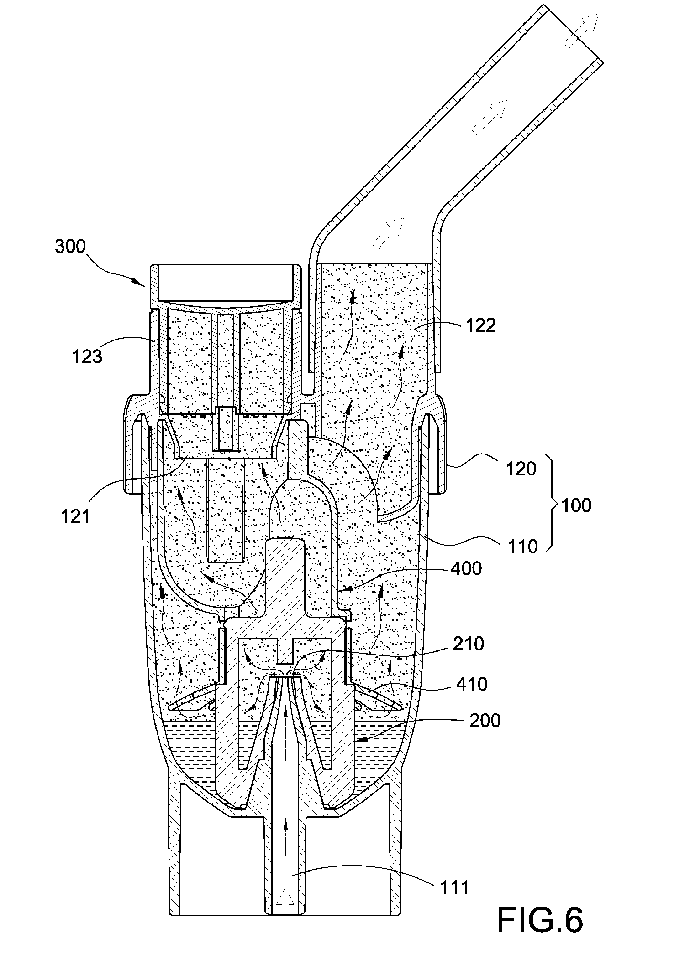

In FIGS. 4 and 6, a liquid medicine is contained at the bottom of the liquid storage container 100, and the bottom of the nozzle 200 and the root portion of the high pressure jet 111 are submerged into the liquid medicine. The adjustable valve 300 can be rotated (as shown in FIG. 4) to cover the port 310 completely by the fixed cover plate 123 of the air inlet 121 of the cap 120. The high pressure jet 111 is connected to an air pressure source, so that high pressure air can flow through the liquid injection opening 210 and produce a negative pressure at the liquid injection opening 210. The medicine is driven by the negative pressure to flow from the bottom of the nozzle 200 and along a gap between the nozzle 200 and the high pressure jet 111 to the liquid injection opening 210, and then pass through the liquid injection opening 210, and the medicine is mixed and atomized with the high pressure air coming from the high pressure jet 111. Since such arrangement only relies on the high pressure air, therefore the nebulization rate is very low. The aerosol medicine further flows through the spray outlet 122 and out from the medical atomizer for the inhale by the patients.

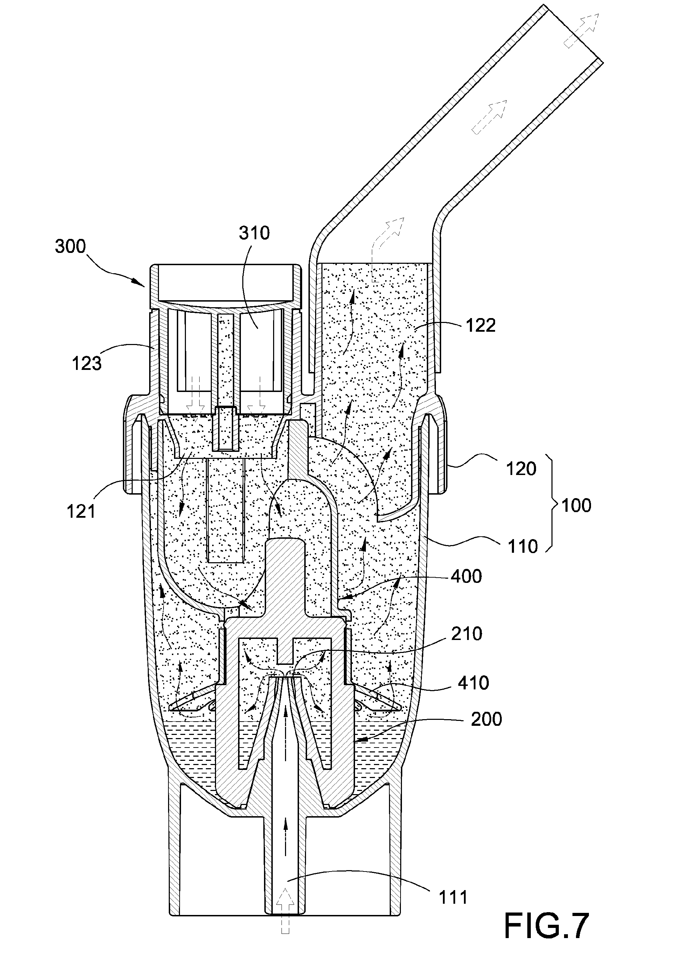

In FIGS. 5 and 7, the adjustable valve 300 is rotated (as shown in FIG. 5) to open the port 310 completely or partially. The high pressure jet 111 is coupled to an air pressure source, so that high pressure air can be injected from the high pressure jet 111 into the medical atomizer of the present invention, wherein the high pressure air flows through the liquid injection opening 210 and produces a negative pressure at the liquid injection opening 210. The medicine is driven by the negative pressure to flow along the gap between the nozzle 200 and the high pressure jet 111 to the liquid injection opening 210. The air outside the medical atomizer is also driven by the negative pressure to pass through the air inlet 121 and through the duct 400 and then enter into the medical atomizer of the present invention, so that both natural air and high pressure air are mixed and atomized outside the opening of the high pressure jet 111. In ,this arrangement with the help of natural air, the nebulization rate can be increased according to the amount of natural air introduced and the aerosol medicine passes through spray outlet 122 and out from the medical atomizer for the inhale by the patients.

In the medicine nebulization process, the medicine is impacted by the high pressure air flow to splatter the medicine, and formed into a water drop shape and attached onto the inner wall of the atomizer easily. As a result, the part of the splattered medicine cannot be used thus causing an unnecessary waste of the medicine. In this preferred embodiment, a baffle 410 is provided for blocking the splattered medicine and minimizing the waste of medicine that is attached onto the inner wall of the medical atomizer and caused during the nebulization process. The splattered medicine is attached onto the baffle 410 first and accumulated there until it flows along the tilted baffle 410 and returns to the bottom of the liquid storage container 100. In addition, the baffle 410 is also used for filtering larger atomized particles and recycling the larger atomized particles for further nebulization, so as to reduce the average size of the atomized particles. The baffle 410 can have notches or holes formed appropriately thereon for adjusting the nebulization quantity and the size of atomized particles.

With the adjustable valve 300 of the air inlet 121, the medical atomizer of the present invention adjusts the flow of natural air entering into the medical atomizer to control the medicine nebulization rate. Compared with the prior art that cannot control the flow of natural air, the present invention can adjust the dosage according to different medication conditions and achieve the effects of avoiding unnecessary waste of medical resources and overcoming the drawbacks of the prior art effectively.

While the invention has been described by means of specific embodiments, numerous modifications and variations could be made thereto by those skilled in the art without departing from the scope and spirit of the invention set forth in the claims.

## Claims

1. A medical atomizer, comprising:
a liquid storage container (100), having an air inlet (121) formed thereon, at least one fixed cover plate (123) installed to the periphery of the air inlet (121), and a high pressure jet (111) installed at the bottom of the liquid storage container (100);
a nozzle (200), installed in the liquid storage container (100), and covered onto the high pressure jet (111); and
an adjustable valve (300), interconnected to the air inlet (121), and having at least one port (310) formed thereon and selectively adjusted and moved to be covered by the fixed cover plate (123).

2. The medical atomizer of claim 1, wherein the liquid storage container (100) includes a main body (110) and a cap (120) installed to the main body (110) and the air inlet (121) is formed on the cap (120).

3. The medical atomizer of claim 1, further comprising a duct (400) with an end interconnected to the air inlet (110).

4. The medical atomizer of claim 3, wherein the duct (400) and the cap (120) are integrally formed as a whole.

5. The medical atomizer of claim 3, wherein the duct (400) has a baffle (410) extended from an outer side of the duct.

6. The medical atomizer of claim 5, wherein the baffle (410) is tilted towards the bottom of the liquid storage container (100).

7. The medical atomizer of claim 3, wherein the duct (410) has another end covered onto the nozzle (200).

8. The medical atomizer of claim 1, wherein the liquid storage container (100) has a spray outlet (122) formed thereon.

9. The medical atomizer of claim 2, wherein the cap (120) has a spray outlet (122) formed thereon.

10. The medical atomizer of claim 1, wherein the nozzle (200) has a liquid injection opening (210) formed at the tip of the nozzle (200).

11. The medical atomizer of claim 2, further comprising a duct (400) with an end interconnected to the air inlet (110).

12. The medical atomizer of claim 11, wherein the duct and the cap (120) are integrally formed as a whole.

13. The medical atomizer of claim 11, wherein the duct (400) has a baffle (410) extended from an outer side of the duct (400).

14. The medical atomizer of claim 13, wherein the baffle (410) is tilted towards the bottom of the liquid storage container (100).

15. The medical atomizer of claim 11, wherein the duct (400) has another end covered onto the nozzle (200).
